(19) Europäisches Patentamt
European Patent Office
Office européen des brevets

(11) **EP 2 306 987 B1**

(12) **EUROPÄISCHE PATENTSCHRIFT**

(45) Veröffentlichungstag und Bekanntmachung des Hinweises auf die Patenterteilung:
**24.01.2018   Patentblatt 2018/04**

(21) Anmeldenummer: **09772483.5**

(22) Anmeldetag: **01.07.2009**

(51) Int Cl.:
***A61K 9/28*** *(2006.01)*

(86) Internationale Anmeldenummer:
**PCT/EP2009/058278**

(87) Internationale Veröffentlichungsnummer:
**WO 2010/000783 (07.01.2010 Gazette 2010/01)**

(54) **VERFAHREN ZUM BESCHICHTEN VON TABLETTEN**

METHOD FOR COATING TABLETS

PROCÉDÉ POUR RECOUVRIR DES COMPRIMÉS

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **02.07.2008   EP 08159504**

(43) Veröffentlichungstag der Anmeldung:
**13.04.2011   Patentblatt 2011/15**

(73) Patentinhaber: **BASF SE**
**67056 Ludwigshafen am Rhein (DE)**

(72) Erfinder:
• **KOLTER, Karl**
  **67117 Limburgerhof (DE)**
• **ROTTMANN, Nils**
  **68169 Mannheim (DE)**
• **ANGEL, Maximilian**
  **67105 Schifferstadt (DE)**

(56) Entgegenhaltungen:
| | |
|---|---|
| **EP-A- 0 572 942** | **EP-A- 1 232 747** |
| **EP-A- 1 929 997** | **GB-A- 1 021 178** |
| **US-A1- 2007 281 012** | **US-A1- 2008 031 950** |

**Beschreibung**

Die vorliegende Erfindung betrifft ein Verfahren zum Beschichten von Substraten, wie z.B. Tabletten, sowie Zusammensetzungen zum Beschichten von festen Substraten in der modernen Arzneiformulierung und Tablettenherstellung ist das Überziehen (Coaten) von festen Darreichungsformen, wie Tabletten, mit unterschiedlichen, filmbildenden Polymeren eine seit Jahren weit verbreitete Technik. Mit der Anwendung des so genannten "Filmcoatings" werden verschiedene Ziele verfolgt. So kann beispielsweise die Farbe und das Aussehen der Darreichungsform verändert werden, es kann ein bitterer Geschmack überdeckt werden oder es wird ein Lichtschutz des Wirkstoffes oder eine Verbesserung der Magensaftresistenz verfolgt.

Wichtige Anforderungen an einen Filmüberzug sind, dass er nicht klebrig ist, gut an der Substratoberfläche haftet, stabil ist gegenüber mechanischer Belastung und keine Rissbildung bei der Lagerung aufweist sowie eine ausgezeichnete Glätte und Glanz aufweist. Zudem sollten sich die Polymere der Filmüberzugslösung schnell und einfach herstellen und in einem Lösemittel (wie Wasser) auflösen lassen. Die Filmüberzugslösung sollte ferner eine geringe Viskosität bei ausreichend hohem Feststoffgehalt aufweisen, um eine gute Verarbeitung, z.B. in einem Sprühverfahren, zu gewährleisten.

Zum Überziehen von Tabletten werden seit Jahren filmbildende Polymere eingesetzt, beispielsweise Cellulosederivate wie Hydroxypropylcellulose, Hydroxypropylmethylcellulose, Methacrylsäure-Copolymere oder auch Polyvinylalkohol-Copolymere. Auch der Einsatz von Polymeren wie Polyethylenglykol und Polyvinylpyrrolidon als filmbildende Komponenten in Beschichtungszusammensetzungen ist bekannt.

So wird in GB 1 021 178 bereits 1964 eine schützende Tablettenbeschichtung für wachsbeschichtete Tabletten aus Polyvinylpyrrolidon und Polyethylenglykol beschrieben. Im beschriebenen Verfahren werden diese Polymere in einem organischen Lösemittel (beispielsweise Aceton, Ethanol oder Trichlorethan) gelöst und auf das feste Substrat gesprüht. Der Feststoffanteil liegt bei diesen Beschichtungen bei 3 bis 18 % , das Verhältnis von Polyethylenglykol zu Polyvinylpyrrolidon in diesen Mischungen beträgt 1:1 bis 9:1. Der in GB 1 021 178 beschriebene Filmüberzug dient in erster Linie dem Schutz der darunter liegenden Wachsbeschichtung und soll deren Lagerstabilität erhöhen. Eine den Glanz steigernde Wirkung des Filmüberzuges wird nicht beschrieben.

US 4,060,598 (1975) beschreibt ein Verfahren zur Herstellung von beschichteten Tabletten, bei dem ein wirkstoffhaltiger Träger mit einem polymeren Überzug versehen wird, wobei die Beschichtung durch das Aufbringen einer wässrigen Zusammensetzung mit einer wasserunlöslichen und einer wasserlöslichen Komponente durchgeführt wird. Die erhaltene Beschichtung bildet eine Matrix aus wasserunlöslichen porösen Harz und einem wasserlöslichen polymeren Material in den Matrixporen. Die Beschichtung dient dazu, den nicht magensaftresistenten Wirkstoff zu schützen und kann zudem in einem wässrigen Beschichtungsverfahren erfolgen. Als Beispiele für die Polymere in der wasserlöslichen Komponente werden in US 4,060,598 unter anderem auch Polyethylenglykol und Polyvinylpyrrolidon genannt. In US 4,060,598 werden auch Zubereitungen genannt, die sowohl Polyethylenglykol als auch Polyvinylpyrrolidon enthalten. Diese wässrigen Zubereitungen enthalten jedoch in jedem Fall auch weitere polymere Komponenten, wie Polyvinylacetat und Polyvinylalkohol sowie Füllstoff und Zucker.

In WO 2006/102446 wird eine mehrfach beschichtete pharmazeutische Zubereitung mit modifiziertem Auflöseverhalten beschrieben. Hierbei wird ein Wirkstoff, beispielsweise 5-Aminosalicylsäure, im Kern der Zubereitung mit einer ersten Beschichtung vor dem Angriff vor Magensäure geschützt. Die zweite, äußere Beschichtung soll die magensaftresistente erste Schicht schützen und kann verschiedene Polymere enthalten, wobei auch Polyvinylpyrrolidon und Polyethylenglykol offenbart werden.

Das Dokument EP-A 0 572 942 offenbart mehrschichtige orale pharmazeutische Zusammensetzungen, die speziell für eine Freisetzung im Bereich des Colons geeignet sind. Die pharmazeutische Zusammensetzung besteht aus einem Kern, der den Wirkstoff und gegebenenfalls Hilfsstoffe enthält, einer Zwischenbeschichtung, welche die Freisetzung des Wirkstoffes in Abhängigkeit vom pH-Wert steuern kann, und eine äußere Schicht, deren Auflösung den Prozess des Quellens und Auflösens der Zwischenschicht startet. Die Beschichtungen können neben einer Reihe von weiteren Bestandteilen beispielsweise Polyethylenglycol und Polyvinylpyrrolidon enthalten.

Das Dokument EP-A 1 232 747 offenbart Tabletten zur verzögerte Freisetzung eines Antidepressivums umfassen einen Kern, enthaltend den Wirkstoffs Bupropion Hydrochlorid, einen Binder und ein Gleitmittel, sowie eine Beschichtung bestehend im Wesentlichen aus einem wasserunlöslichen filmformenden Polymer, einem Weichmacher und einem wasserlöslichen Polymer.

Das Dokument EP-A 1 929 997 beschreibt pharmazeutische Darreichungsformen für das Antiepileptikum Oxcarbazepin, wobei der Wirkstoff eine definierten Partikelgröße aufweist. Die Herstellung der Darreichungsform erfolgt unter Verwendung einer Suspension des Wirkstoffs und eines hydrophilen Polymers in einem Lösungsmittel. Unter anderem sind Polyvinylpyrrolidon, Polyethylenglycol und Hydroxyethylzellulose als mögliche hydrophile Polymere genannt.

Das Dokument US 2008/0031950 beschreibt eine mehrschichtige pharmazeutische Zusammensetzung enthaltend einen Kern, welcher den Wirkstoff Tramadol langsam freisetzt (slow release matrix), und eine Beschichtung enthaltend ein NSAID (nonsteroidal anti-inflammatory drug), welche den Wirkstoff unverzüglich freisetzt. Zwischen diesen beiden Bereichen ist eine Beschichtung zur kontrollierten Freisetzung (slow release coat) des Wirkstoffes Tramadol vorgesehen enthaltend ein wasserunlösliches, wasserpermeables Polymer, insbesondere Ethylcellulose und weitere polymere Hilfsstoffe, z.B. wasserlösliche Polymere wie Polyvinylpyrrolidon, Hydroxypropylmethylcellulose oder Polyethylenglykol.

Die erfindungsgemäße Zusammensetzung soll es ermöglichen ein Substrat, d.h. eine vorzugsweise feste Grundlage (Träger), vorzugsweise eine pharmazeutische, kosmetische oder agrochemische Darreichungsform, ein Saatgut, Nahrungsergänzungsmittel und / oder ein Lebensmittel zu beschichten. Die Zusammensetzung besteht bevorzugt aus einer wässrigen Lösung von Polyethylenglykol (Komponente A) mit einem gewichtsmittleren Molekulargewicht $M_w$ im Bereich von 1.500 bis 20.000 g/mol und einem Polyvinylpyrrolidon (Komponente B) mit einem K-Wert im Bereich von 12 bis 90, sowie optional weiteren, nicht polymeren Hilfsmitteln (Komponente C). Weiterhin soll die Erfindung ein verbessertes Verfahren zur Herstellung eines beschichteten, in der Regel (festen) Substrates bereitstellen.

Wird als Substrat eine pharmazeutische Darreichungsform z.B. eine Tablette verwendet, so kann diese einen oder mehrere Wirkstoffe (wie handelsübliche Arzneimittel-Wirkstoffe) enthalten.

Die bekannten Polymerzusammensetzungen haben beim Aufbringen auf Substraten (wie Tabletten) den Nachteil, häufig eine matte, unansehnliche und rauhe Oberfläche zu erzeugen. Um solchen Substraten ein ansehnlicheres, glänzendes Aussehen zu verleihen, und beispielsweise einem Patienten ein leichteres Schlucken der beschichteten Tabletten zu ermöglichen, sollen Substrate mit einem (gegebenenfalls zusätzlichen) glatten und glänzenden Film überzogen werden. Um dieses Ziel zu erreichen, soll eine den Glanz erzeugende bzw. verbessernde Beschichtung bereitgestellt und auf das Substrat aufgetragen werden. Das Verfahren des Aufbringens der Zusammensetzung sollte einfach und bevorzugt mit einer wässrigen Lösung durchzuführen sein. Die Beschichtung kann auch im Anschluss an einen klassischen Filmcoatingprozess erfolgen.

Es wurde überraschenderweise gefunden, dass das Beschichten (Überziehen) von Substraten, insbesondere stumpfen und rauhen Substraten (wie Filmtabletten), mit einer wässrigen Lösung aus speziellen, wasserlöslichen Polyethylenglykolen (PEG) und Polyvinylpyrrolidonen (PVP) eine deutliche Glanzverbesserung der Substrate (z.B. Tabletten) bewirkt.

Die erfindungsgemäßen Zusammensetzungen lassen sich sehr einfach und schnell durch Lösen der Komponenten in einem Lösemittel (insbesondere Wasser) herstellen, besitzen niedrige Viskositäten und lassen sich auch in höherer Feststoffkonzentration mit einer guten Aufbringungsrate z.B. in herkömmlichen Coating-Verfahren auf feste Substrate aufbringen. Die erfindungsgemäßen Filmüberzüge zeichnen sich neben hervorragendem Glanz und Glätte durch niedrige Klebrigkeiten und gute Lagerstabilität aus.

Die erfindungsgemäßen Zusammensetzungen bewirken eine deutliche Glanzsteigerung von z.B. bis zu 285%. Die Kombinationen aus Polyvinylpyrrolidon und Polyethylenglykol bewirken, wie nachfolgend gezeigt wird, eine deutlich höhere Glanzverbesserung als Lösungen der jeweiligen Einzelkomponenten.

Die erfindungsgemäße Zusammensetzung zur Beschichtung von festen Substraten (S), wie Arzneimitteltabletten, besteht aus:

a) 5-20 Gew.-% eines wasserlöslichen Polyethylenglykol mit einem gewichtsmittleren Molekulargewicht im Bereich von 1.500 bis 10.000 g/mol (Komponente A),

b) 0,5-5 Gew.-% eines wasserlöslichen Polyvinylpyrolidons mit einem K-Wert nach Fikentscher im Bereich von 12 bis 90 (Komponente B),

c) 65-95 Gew.-% Wasser als Lösemittel (L), sowie

d) 0-10 Gew.-% eines oder mehrerer nicht polymerer Hilfsmittel (Komponente C),

wobei es sich bei der Zusammensetzung um eine wässrige Lösung des Polyethylenglykol, des Polyvinylpyrrolidons und optional der nicht polymeren Hilfsmittel handelt, und wobei die Zusammensetzung einen Polymergehalt im Bereich von 5 bis 20 Gew.-% aufweist und Polyethylenglykol und Polyvinylpyrrolidon in einem Gewichtsverhältnis von 15:1 bis 7:1 vorliegen.

Eine weitere Ausführungsform betrifft eine Zusammensetzung zur Beschichtung von festen Substraten bestehend aus:

a) 5-20 Gew.-% eines wasserlöslichen Polyethylenglykol mit einem gewichtsmittleren Molekulargewicht im Bereich von 4.000 bis 10.000 g/mol (Komponente A)

b) 0,5-5 Gew.-% eines wasserlöslichen Polyvinylpyrolidons mit einem K-Wert nach Fikentscher im Bereich von 12 bis 30 (Komponente B)

c) 75-95 Gew.-% Wasser als Lösemittel (L).

Die Komponente B wird vorzugsweise ausgewählt aus einem oder mehreren Polyvinylpyrrolidonen mit einem K-Wert von 12 bis 90, vorzugsweise von 12, 17, 30, 90, insbesondere 12, 17 oder 30.

Eine weitere Ausführungsform betrifft eine Zusammensetzung zur Beschichtung von festen Substraten, wobei die Lösung einen Polymergehalt im Bereich von 5 bis 20, insbesondere 7 bis 15 Gew.-% aufweist, und Polyethylenglykol und Polyvinylpyrrolidon in einem Gewichtsverhältnis von 15:1 bis 7:1, insbesondere jedoch etwa 9:1 vorliegen.

Eine Zusammensetzung zur Beschichtung von festen Substraten hat sich besonders bewährt, die enthält, beziehungsweise besteht aus:

a) 8-10 Gew.-% eines wasserlöslichen Polyethylenglykol mit einem gewichtsmittleren Molekulargewicht von 6000 g/mol (PEG 6000) (Komponente A),

b) 0,5-2 Gew.-% eines wasserlöslichen Polyvinylpyrolidons mit einem K-Wert nach Fikentscher von 17 (Komponente B), sowie

c) 88-95 Gew.-% Wasser, insbesondere 92,5 bis 95 Gew.-% Wasser.

Die Erfindung betrifft auch ein Verfahren zur Herstellung beschichteter fester Substrate (wie Tabletten), bei dem man auf ein festes Substrat (S) mindestens eine Zusammensetzung wie oben beschrieben aufbringt.

Dieses Verfahren kann vorzugsweise die folgenden Schritte beinhalten:

a) Herstellen einer Beschichtungslösung durch Vermischen der Komponenten A und B und des Lösemittels L,

b) Aufbringen der Beschichtungslösung mittels eines geeigneten Verfahrens auf das Substrat (S),

c) gegebenenfalls Trocknen des beschichteten Substrats durch das Aufbringen von Gas bei einer Temperatur im Bereich von 20°C bis 80°C, sowie

d) gegebenenfalls Polieren des beschichteten und getrockneten Substrats.

Die Erfindung betrifft auch ein Verfahren zur Herstellung beschichteter fester Substrate bei dem das Substrat (S) ausgewählt ist aus: festen pharmazeutischen, kosmetischen und agrochemischen Darreichungsformen, Saatgut, Nahrungsergänzungsmitteln und Lebensmitteln.

Die Erfindung betrifft auch ein Verfahren zur Herstellung beschichteter fester Substrate bei dem das Substrat (S) aus einer festen pharmazeutischen Darreichungsform (z.B. einer Tablette) besteht, welche bereits mit mindestens einer polymeren Filmbeschichtung versehen ist.

Gegenstand der Erfindung ist auch die Verwendung einer Zusammensetzung zur Herstellung von beschichteten pharmazeutischen, kosmetischen oder agrochemischen Darreichungsformen, von beschichtetem Saatgut, von beschichteten Nahrungsergänzungsmitteln oder von beschichteten Lebensmitteln.

Ein weiterer Aspekt der Erfindung ist das beschichtete Substrat, das beschichtet ist mit einer Zusammensetzung wie oben beschrieben. Dieses beschichtete Substrat kann basieren z.B. auf einem Substrat (S) ausgewählt aus: pharmazeutischen, kosmetischen und agrochemischen Darreichungsformen, Saatgut, Nahrungsergänzungsmitteln und Lebensmitteln.

Als Komponente A werden wasserlösliche Polyethylenglykole (PEG) mit einem mittleren Molekulargewicht $M_w$ im Bereich von 1.500 bis 10.000 g/mol eingesetzt. Vorzugsweise werden folgende Polyethylenglykole eingesetzt:

Polyethylenglykol mit einem mittleren Molekulargewicht $M_w$ von 1.500 g/mol (PEG 1.500)
Polyethylenglykol mit einem mittleren Molekulargewicht $M_w$ von 2.000 g/mol (PEG 2.000)
Polyethylenglykol mit einem mittleren Molekulargewicht $M_w$ von 4.000 g/mol (PEG 4.000)
Polyethylenglykol mit einem mittleren Molekulargewicht $M_w$ von 6.000 g/mol (PEG 6.000)

Besonders bevorzugt wird das Polyethylenglykol PEG 6.000 eingesetzt.

Als Komponente B können insbesondere Polyvinylpyrrolidone (PVP) mit einem K-Wert nach Fikentscher im Bereich von 12 bis 90, vorzugsweise von 17 bis 30, besonders bevorzugt mit K=17 eingesetzt werden.

Unter Polyvinylpyrrolidonen (PVP) werden polymere Verbindungen verstanden, welche einen Anteil an Vinylpyrrolidon-Monomereinheiten von mindestens 50 % und gegebenenfalls weitere Monomereinheiten (wie beispielsweise Vinylacetat (VAc)) aufweisen. Bevorzugt sind Polyvinylpyrrolidone, die nahezu vollständig aus Vinylpyrrolidon-Einheiten aufgebaut sind.

Bevorzugt sind weiterhin Zusammensetzungen mit Polyvinylpyrrolidonen (PVP) mit einem K-Wert von 12, 17, 30, 90. Folgende Polyvinylpyrrolidone werden bevorzugt eingesetzt:

| | |
|---|---|
| Kollidon® 12 (BASF, Ludwigshafen DE) | PVP mit K=12 |
| Kollidon® 17PF (BASF, Ludwigshafen DE) | PVP mit K=17 |
| Kollidon® 30 (BASF, Ludwigshafen DE) | PVP mit K=30 |
| Kollidon® 90F(BASF, Ludwigshafen DE) | PVP mit K=90. |

Es können auch Copolymere aus Vinylpyrrolidon und Vinylacetat verwendet werden, z.B. das handelsübliche Kollidon® VA64 der BASF (PVP-Vinylacetat (VAc) Copolymer).

Besonders bevorzugt werden folgende Polyvinylpyrrolidone als Komponente B in den erfindungsgemäßen Zusammensetzungen eingesetzt:

| | |
|---|---|
| Kollidon® 17PF (BASF, Ludwigshafen DE) | PVP mit K=17 |
| Kollidon® 30 (BASF, Ludwigshafen DE) | PVP mit K=30. |

Die Polyvinylpyrrolidone mit K=17 werden ganz besonders bevorzugt eingesetzt.

Das mittlere Molekulargewicht der Polyvinylpyrrolidon-Komponente B wird in der Praxis bevorzugt durch den K-Wert nach Fikentscher angegeben, der sich relativ einfach durch Viskositätsmessungen von den entsprechenden verdünnten Polymerlösungen bestimmen lässt. Der K-Wert berechnet sich nach der Fikentscher-Gleichung aus der relativen Viskosität $\eta_r$ :

$$K = 1000 \cdot k = 1000 \cdot \frac{1{,}5 \lg \eta_r - 1 \pm \sqrt{1 + \left(\frac{2}{c} + 2 + 1{,}5 \lg \eta_r\right) \cdot 1{,}5 \lg \eta_r}}{150 + 300 \cdot c}$$

mit:

$\eta_r$ = dynamische Viskosität der Lösung/dynamische Viskosität des Lösemittels

c = Massenkonzentration an Polymer in der Lösung in g/cm$^3$

Der K-Wert nach Fikentscher stellt somit ein Maß für das viskositätsmittlere Molekulargewicht dar.

Im Folgenden sind die entsprechenden Molekulargewichtsbereiche der eingesetzten Polyvinylpyrrolidone (PVP) bezogen auf das gewichtsmittlere Molekulargewicht $M_w$, welches etwa durch Lichtstreumessungen ermittelt werden kann, angegeben

| | |
|---|---|
| PVP mit einem K-Wert von 12: | $M_w$ 2.000 bis 3.000 g/mol |
| PVP mit einem K-Wert von 17: | $M_w$ 7.000 bis 11.000 g/mol |
| PVP mit einem K-Wert von 30: | $M_w$ 44.000 bis 54.000 g/mol |
| PVP mit einem K-Wert von 90: | $M_w$ 1.000.000 bis 1.500.000 g/mol. |

Als Lösemittel (L) wird Wasser, insbesondere VE Wasser (voll entsalztes Wasser) verwendet.

Optional können die erfindungsgemäßen Zusammensetzungen als Komponente C nicht polymere Hilfsstoffe enthalten, wie sie als Bestandteile für Tablettenüberzüge üblich sind. Als Hilfsstoffe können insbesondere eine oder mehrere der nachfolgenden Komponenten ausgewählt werden:

a) Farbgebende Komponenten:

Farbpigmente wie z.B. Eisenoxide und Farbstoffe in wasserlöslicher oder wasserunlöslicher Form, z.B. Chinolingelblack, Tartrazinlack, Gelborangelack, FD&C Gelb-Aluminiumlack, Cochenillerotlack, Erythrosinlack, Azorubinlack, Indigotinlack, Erythrosin, Brillantschwarz, Patentblau, Brilliantblau, Cochenillerot, Gelborange, Amaranth, FD&C Blau Nr. 1, Indigotin, Betacarotin sowie Perlglanzpigmente

b) Weißpigmente zur Erhöhung der Deckkraft des Überzuges, z.B. Titandioxid, Talkum; Mica; Calciumcarbonat

c) Antiklebemittel z.B. Talkum, Magnesiumstearat, Glycerolmonostearat;

d) Füllstoffe wie z. B. Calciumhydrogenphosphate;

e) Schaumhemmende bzw. zerstörende Stoffe wie z.B. Simethicon, Octanol;

f) Tenside zur Verbesserung des Benetzungsverhaltens und der Spreitung, z.B. Natriumlaurylsulfat, Sorbitanfettsäureester oder ethoxilierte Sorbitanfettsäureester, ethoxylierte Ester von hydriertem Rizinusöl oder ethoxilierte Fettsäureester, Natriumdioctylsulfosuccinat;

g) den pH-Wert regulierende Substanzen und Puffer wie z.B. Natriumcitrat, Citronensäure, Phosphatpuffer, Acetatpuffer:

h) Weichmacher;
i) Schutzkolloide;
j) Geschmacksstoffe und / oder Geruchsstoffe.

Die erfindungsgemäßen Zusammensetzungen haben den Vorteil, dass sie in der Regel keine weiteren Hilfsmittel wie Weichmacher oder Tenside benötigen, welche negative Begleiterscheinungen verursachen können. In einer bevorzugten Ausführungsform sind keine weiteren Hilfsstoffe (Komponente C) in den Zusammensetzungen enthalten.

Gegenstand der Erfindung ist auch die Trockenmischung der Komponenten A und B und gegebenenfalls C oder ein aus dieser Trockenmischung hergestelltes Granulat der Komponenten A und B (und gegebenenfalls C), welches dann zur Herstellung der erfindungsgemäßen glanzgebenden Filmüberzugslösungen verwendet werden kann.
Die Erfindung betrifft auch ein Kit, bestehend aus den einzelnen Komponenten der Zusammensetzung, das dann vom Benutzer zur Herstellung der Beschichtungszusammensetzung verwendet wird.

Das Verfahren zur Herstellung beschichteter Substrate kann beispielsweise die folgenden konkreten Schritte beinhalten:

a) Herstellen der Beschichtungslösung durch Zugabe unter Rühren der Komponenten A und B und ggf. C im Lösemittel L,
oder optional Mischen der festen Komponenten A und B und ggf. C und anschließendes Lösen der festen Mischung im Lösemittel L,
oder optional Lösen bzw. Dispergieren der Komponenten A, B und ggf. C in Teilen des Lösemittels L und anschließendes Vereinigen der Lösungen,

b) Aufbringen der Beschichtungslösung mittels eines geeigneten Sprühverfahrens auf das Substrat

c) Trocknung der Beschichtung, wobei der Filmüberzug durch das Einbringen von Luft mit einer Temperatur im Bereich von 30°C bis 80°C sukzessive getrocknet wird,

c) Optionale Durchführung eines Polierschritts mit den beschichteten und getrockneten Substraten.

Zu Schritt a)

Vorzugsweise erfolgt das Herstellen der Zusammensetzungen (Filmüberzugslösungen) durch langsame Zugabe unter Rühren und vollständiges Lösen der Komponenten A und B sowie gegebenenfalls C im Lösemittel L. Eine Erwärmung der Lösung während der Herstellung ist in der Regel nicht erforderlich.

In einer weiterhin bevorzugten Ausführungsform werden zunächst die Komponenten A und B sowie gegebenenfalls C gemischt und evtl. granuliert werden. Die Herstellung der Zusammensetzung erfolgt dann durch Zugabe der festen Mischung unter Rühren zu dem Lösemittel L.

Zu Schritt b)

Mit dem erfindungsgemäße Verfahren zur Beschichtung lassen sich prinzipiell alle festen Substrate mit einem Polymerfilm überziehen. Beschichtet werden können z.B. feste pharmazeutische, kosmetische und agrochemische Darreichungsformen, Saatgut, Nahrungsergänzungsmittel und Lebensmittel. Die zu überziehende pharmazeutische Darreichungsform kann beispielsweise als Tablette, Kapsel, Extrudat, Pellet, Granulat, Kristall oder Pulver vorliegen.

Es ist weiterhin möglich feste Substrate mit unterschiedlich geformten Oberflächen zu beschichten, wobei die Oberfläche des festen Substrates beispielsweise gewölbt, konvex oder konkav ausgebildet sein kann. So kann durch die erfindungsgemäße Zusammensetzung auch bei festen Substraten mit Gravur eine nahezu vollständige Auskleidung der Gravur mit einer glanzgebenden Beschichtung erzielt werden. Die festen Substrate können unterschiedliche Formen, wie etwa rund, vieleckig, "oblong" oder "football-shape", aufweisen.

In einer bevorzugten Ausführungsform ist das zu überziehende Substrat (S) ausgewählt aus festen pharmazeutischen, kosmetischen, agrochemischen Darreichungsformen, Saatgut, Nahrungsergänzungsmitteln und Lebensmitteln.

Besonders bevorzugt erfolgt die Beschichtung auf festen Substraten, die aus einer pharmazeutischen Darreichungsform, wie einer Tablette bestehen, welche bereits mit mindestens einer polymeren Beschichtung (Basis-Beschichtung) ausgestattet sind.

Die Anwendung des erfindungsgemäßen Verfahrens ist dabei einfach durchzuführen, da der glanzgebende Filmüberzug direkt nach der Basis-Beschichtung oder nach den Basis-Beschichtungen aufgetragen werden kann. Es ist keine zusätzliche Vorrichtung erforderlich und die Applikationsdauer ist im Vergleich zum Basis-Beschichtungsprozess kurz.

Dabei können die Basis-Beschichtungen etwa dazu dienen, den Wirkstoff zu schützen, z.B. vor Wasser, Sauerstoff, Protonen, chemischen Bestandteilen des Überzuges sowie vor Komponenten des Magen- und Darminhaltes oder zur Einfärbung der pharmazeutischen Darreichungsform.

Im Kern der pharmazeutischen Darreichungsform (oder auf der Oberfläche des Substrates) können Wirkstoffe verschiedener Indikationsgebiete eingesetzt werden, beispielsweise pharmazeutische Wirkstoffe (Humanarzneistoffe sowie Tierarzneistoffe), Vitamine, Carotinoide, "Nutraceuticals", Nahrungsergänzungsstoffe, Mineralstoffe und Mikro-Nährstoffe. Die Wirkstoffe können einzeln oder in Kombination eingesetzt werden und unterschiedliche pharmakologische und physikochemische Eigenschaften wie Lipophilie, Löslichkeit, Korngröße, Kornstruktur und Oberfläche besitzen.

Das Aufbringen der erfindungsgemäßen Beschichtung geschieht bevorzugt in einem Sprühverfahren und kann in den dazu geeigneten Coatingeinrichtungen durchgeführt werden. Als Beispiele werden Horizontal-Trommelcoater, Wirbelschichtcoater, Tauschwertcoater und Dragierkessel genannt. Für das Zerstäuben der Polymerlösung wird beispielsweise eine Zweistoffdüse verwendet.

Hierbei ist es auch möglich, die erfindungsgemäße Beschichtung direkt im Anschluss an eine erste Beschichtung des Substrats, beispielsweise einer pharmazeutischen Darreichungsform, und in derselben Beschichtungsvorrichtung durchzuführen. Die erfindungsgemäße Lösung konnte auf allen getesteten Farben und auf allen getesteten, filmbildenden Polymeren angewendet werden.

Es können z.B. Sprühlösungen mit einem Feststoffgehalt von bis zu 30 Gew.-% appliziert werden. Die optimalen Auftragsmengen liegen im Bereich von 0,2 bis 2 mg/cm$^2$.

Zu Schritt c)

Die Trocken- und Abkühlzeit liegt vorzugsweise im Bereich von 2 bis 20 Minuten bei einer (Produkt- oder Kernbett-)Temperatur im Bereich von 20 bis 50°, insbesondere 30 bis 50°C. Der Sprühdruck liegt in der Regel im Bereich von 1 bis 3 bar.

Zu Schritt d)

An die Herstellung der Beschichtung kann sich optional ein Polierschritt anschließen. Die Nachbehandlung (Polieren) bei niedriger Drehzahl und geringer Zuluft-Temperatur bewirkt bei einigen Rezepturen eine weitere Verbesserung des Glanzes.

In einer bevorzugten Ausführungsform der Erfindung wird eine feste pharmazeutische Darreichungsform, welche bereits mit mindestens einer Basis-Beschichtung bestehend aus einem Polyethylenglykol/Polyvinylalkohol-Copolmeren beschichtet ist, mit einer Zusammensetzung bestehend aus:

9 Gew.-% Polyethylenglykol mit einem gewichtsmittleren Molekulargewicht von 6000 g/mol (PEG 6000) (Komponente A),

1 Gew.-% des wasserlöslichen Polyvinylpyrolidons mit einem K-Wert nach Fikentscher von 17 (PVP-17) (Komponente B), sowie

90 Gew.-% Wasser (L)

in einem Sprühverfahren direkt anschließend an eine vorherige Beschichtung beschichtet.

Die folgenden Beispiele zeigen die glanzerzeugende Wirkung verschiedener erfindungsgemäßer Zusammensetzungen auf bereits filmbeschichtete Tabletten mit unterschiedlichen Formen, Farben und verschiedenen Basis-Beschichtungen.

**Beispiele**

Es wurden die in Tabelle 1 beschriebenen Komponenten eingesetzt:

Tabelle 1: Einsatzstoffe

| Komponente | Bezeichnung | Beschreibung |
|---|---|---|
| Placebokerne | Placebokerne | 99,5% Ludipress® LCE (BASF, Ludwigshafen, DE); Mischung aus Lactose und Kollidon® 30 (BASF, Ludwigshafen, DE) als Binder<br>0,5% Mg-Stearat |
| VE-Wasser | VE-Wasser | Vollentsalztes Wasser |
| A | PEG 1.500 | Polyethylenglykol mit $M_w$=1.500 g/mol |
| A | PEG 2.000 | Polyethylenglykol mit $M_w$=2.000 g/mol |
| A | PEG 4.000 | Polyethylenglykol mit $M_w$=4.000 g/mol |
| A | PEG 6.000 | Polyethylenglykol mit $M_w$=6.000 g/mol |
| A | PEG 20.000 | Polyethylenglykol mit $M_w$=20.000 g/mol |
| B | PVP K 12 | Polyvinylpyrolidon mit K=12<br>Handelsname Kollidon® 12 (BASF, Ludwigshafen DE) |
| B | PVP K 17 | Polyvinylpyrolidon mit K=17<br>Handelsname Kollidon® 17PF (BASF, Ludwigshafen DE) |
| B | PVP K 30 | Polyvinylpyrolidon mit K=30<br>Handelsname Kollidon® 30 (BASF, Ludwigshafen DE) |
| B | PVP K 90 | Polyvinylpyrolidon mit K=90 Handelsname Kollidon® 90F® (BASF, Ludwigshafen DE) |
| B | PVP-VAc-Copolymer | Handelsname Kollidon® VA64® (BASF, Ludwigshafen DE) |
| C | Hydroxypropylmethylcellulose (3 mPas) | Pharmacoat® 603 (Shin-etsu) |
| Basis-Coating -Typ I | PEG-PVA-Copolymer - weiß | Polyethylenglykol-Polyvinylalkohol-Copolymer Granulat mit weißem Pigment (Titandioxid, Kadin)<br>Kollicoat® IR White |
| Basis-Coating - Typ II | PEG-PVA-Copolymer - gelb | Polyethylenglykol-Polyvinylalkohol Copolymer Granulat mit gelbem Farbstoff<br>Kollicoat® IR White + Sicovit® Gelborange (5 %) |
| Basis-Coating - Typ III | PEG-PVA-Copolymer - rot | Polyethylenglykol-Polyvinylalkohol Copolymer Granulat mit rotem Farbpigment<br>Kollicoat® IR White + Sicovit® Rot (5%) |
| Basis-Coating - Typ IV | PEG-PVA-Copolymer - carmin | Polyethylenglykol-Polyvinylalkohol Copolymer Granulat mit carmin-farbigem Farbpigment<br>Kollicoat® IR White + Carmine (5 %) |

(fortgesetzt)

| Komponente | Bezeichnung | Beschreibung |
|---|---|---|
| Basis-Coating - Typ V | PEG-PVA-Copolymer - brilliant blue | Polyethylenglykol-Polyvinylalkohol Copolymer Granulat mit brillant bluefarbigem Farbpigment<br>Kollicoat® IR White + Brilliant Blue (5 %) |
| Basis-Coating - Typ VI | PEG-PVA-Copolymer - schwarz | Polyethylenglykol-Polyvinylalkohol Copolymer Granulat mit schwarzem Farbpigment<br>Kollicoat® IR + Sicovit® Schwarz (5%) |
| Basis-Coating - Typ VII | PEG-PVA-Copolymer /PVA - weiß | Polyethylenglykol-Polyvinylalkohol Copolymer / Polyvinylalkohol mit weißem und roten Farbpigment<br>Kollicoat® Protect + Kadin, Titandioxid und Sicovit® Rot |
| Basis-Coating - Typ VIII | Hydroxypropylmethylcellulose (HPMC), weiß | Hydroxypropylmethylcellulose mit weißem Pigment (Kadin, Titandioxid) |
| Rotes Farbpigment | Eisenoxid | Eisenoxid, Handelsname Sicovit® Rot (BASF, Ludwigshafen DE) |
| Gelber Farbstoff | Azofarbstoff, C.I. Acid Yellow 3 (E 110) | Handelsname Sicovit® Gelborange (BASF, Ludwigshafen DE) |
| Weißpigment | Kadin | Kadin |
| Weißpigment | Titandioxid | Titandioxid |

Alle Prozentangaben beziehen sich, soweit nichts anderes angegeben ist, auf Gewichtsprozent, K bezeichnet den K-Wert nach Fikentscher, $M_w$ bezeichnet das gewichtsmittlere Molekulargewicht.

Es wurden die folgenden Geräte eingesetzt:

- Automatische Tabletten-Beschichtungs-Apparatur; Horizontal-Trommelcoater vom Typ Accela Cota 24" (Fa. Manesty, Liverpool, GB)

- High-Shear-Mischer, Ultra Turrax (Hersteller IKA-Werke GmbH, Staufen DE)

- Flügelblattrührer mit Antrieb

- Magnetrührer

- Glasgeräte.

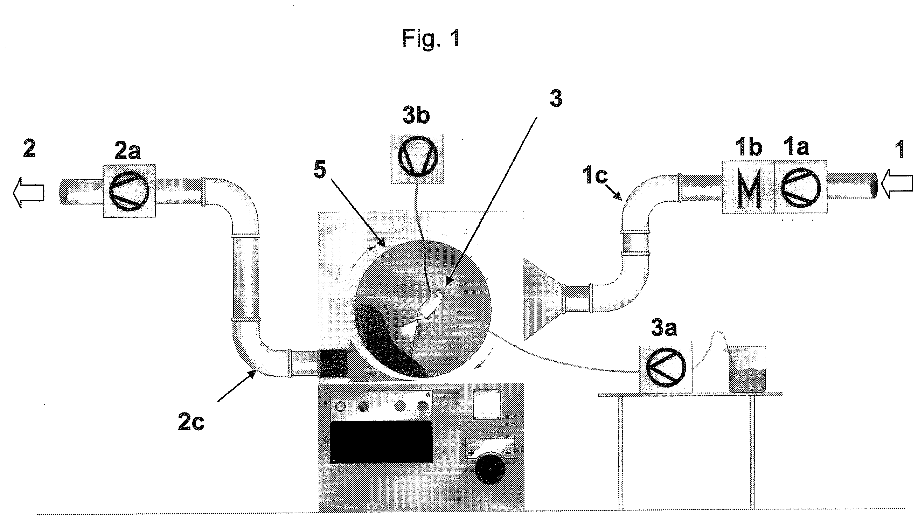

Eine Skizze der Beschichtungs-Apparatur (Accela Cota 24", Manesty), die vor Durchführung des Verfahrens geeignet ist, wird in Fig. 1 wiedergegeben. Im einströmenden Luftstrom (1) werden die Zuluft-Menge (1a), die Zuluft-Temperatur (1b) und die Zuluft-Feuchte (1c) bestimmt. Im austretenden Luftstrom (2) wird die Abluft-Menge (2a) und die Abluft-Feuchte (2c) bestimmt. Des Weiteren ist die Sprühdüse (3) abgebildet, wobei Sprührate (3a) und Sprühdruck (3b) bestimmt werden können. Der Beschichtungskessel (4) rotiert mit der Kesseldrehzahl. Im Folgenden ist eine Liste der in Fig. 1 abgebildeten Teile und Beschichtungsparameter wiedergegeben:

(1) Zuluft
(1a) Zuluft-Menge
(1b) Zuluft-Temperatur
(1c) Zuluft-Feuchte
(2) Abluft
(2a) Abluft-Menge
(2c) Abluft-Feuchte
(3) Sprühdüse
(3a) Sprührate
(3b) Sprühdruck

(4) Beschichtungskessel.

Die Glanzmessungen erfolgten mit einem üblichen Glanzmessgerät für gewölbte Oberflächen, z.B. vom Typ 400 Novo-Curve™ (Hersteller: Elcometer Instruments, Manchester GB). Die Messungen wurden mit einem Messwinkel von 60° bei Raumtemperatur (20°) durchgeführt. Der Glanzwert wird dimensionslos als Gloss Units [GU] angegeben. Es wurde jeweils ein Mittelwert aus 10 Messungen an 10 verschiedenen Tabletten der Probe gebildet.

**Beispiel 1** - Herstellen von Beschichtungslösungen (Filmüberzugslösungen) für die Basis-Beschichtung

a) Basis-Coating Typ II mit wasserlöslichem Farbstoff

Die für die Basis-Beschichtung verwendeten Mischungen wurden durch Lösen von Kollicoat® IR White und dem Farbstoff Sicovit® Gelborange in VE-Wasser hergestellt. Dazu wurden die Komponenten zusammen unter Rühren mit einem Blattrührer in VE-Wasser gelöst bzw. dispergiert. Die Lösung wurde über Nacht auf einem Magnetrührer bei niedriger Drehzahl entlüftet. Der Polymerfilm bestand zu 92,9% aus Kollicoat® IR B08/0345PC
White und zu 7,1% aus dem Farbstoff Sicovit® Gelborange. Die Lösung besaß insgesamt einen Feststoffgehalt von 20%.

b) Basis-Coating Typ III - VI mit wasserunlöslichem Farbstoff

Kollicoat® IR White wurde unter Rühren in einem Teil des VE-Wassers dispergiert. Seperat wurde das entsprechenden Farbpigments mit einem High-Shear-Mischer (Ultra-Turrax) in einem anderen Teil des VE Wassers dispergiert. Die beiden Mischungen wurden unter Rühren zusammengeführt und über Nacht durch leichtes Rühren entgast.

c) Basis-Coating Typ VII und VII mit wasserunlöslichem Farbstoff

Die für die Basis-Beschichtung verwendete Polymerlösung wurde durch Lösen des Polymers Typ VII bzw. Typ VIII und dispergieren der Farbpigmente Titandioxid, Kadin, Eisenoxid (Sicovit® Rot) in VE-Wasser hergestellt. Dazu wurde das Polymer in einem Teil des Wassers gelöst und die Pigmente in einem anderen Teil des Wassers mit einem High-Shear-Mischer (Ultra Turrax) dispergiert. Die beiden Mischungen wurden unter Rühren mit einem Blattrührer zusammengeführt. Die Lösung wurde über Nacht auf einem Magnetrührer bei niedriger Drehzahl entlüftet.
Der Polymerfilm Typ VII bestand zu 25 % aus Kollicoat® Protect, 67 % Kadin, 2 %Titandioxid und zu 6 % aus Eisenoxid (Sicovit® Rot). Die Lösung besaß insgesamt einen Feststoffgehalt von 25 %.
Der Polymerfilm Typ VIII bestand zu 75 % aus Hydroxypropylmethylcellulose, 10 % Kadin, 5 %Titandioxid und 10 % Polyethylenglykol 6000. Die Lösung besaß insgesamt einen Feststoffgehalt von 12 %.

**Beispiel 2** - Auftrag der Basis-Beschichtung

Die in den Beispielen 1 a) bis c) beschriebenen Lösungen bzw. Dispersionen wurden separat in einer Tabletten-Beschichtungs-Apparatur (24" Accela Cota) unter den unten aufgeführten Auftragsbedingungen auf Placebokerne aus 99,5 % Ludipress LCE® (BASF) und 0,5 % Magnesium-Stearat aufgetragen. Als Sprühdüse wurde eine übliche Düse (Modell 930, Hersteller: Düsen-Schlick, Untersiemau DE) mit einer 1 mm-Bohrung verwendet. Eine Skizze der Beschichtungs-Apparatur (Manesty 24" Accela Cota) ist in Fig. 1 wiedergegeben.

| Bedingungen Auftragsprozess der Basis-Beschichtung: | |
|---|---|
| Sprühdruck | 2,0 bar |
| Formierluftdruck | 1,0 bar |
| Kesseldrehzahl | 15 UpM |
| Zuluft-Menge | 60 L/s |
| Abluft-Menge | 110 L/s |
| Zuluft-Temperatur | 62°C |
| Abluft-Temperatur | 45°C |
| Produkttemperatur | 37°C |
| Sprührate | 25 g/min |
| Trocken-/Abkühlzeit | 10 min |
| Auftragsmenge | 4,5 mg/cm$^2$ |

(fortgesetzt)

| Batchgröße | 5 kg |
|---|---|

Im Anschluss an diesen Prozess wurde eine Probe von mindestens 10 Tabletten zur Glanzmessung entnommen. Die Glanzmessung an den Filmtabletten wurde mit einem üblichen optischen Messgerät (Typ 400 Novo-Curve™) bei einem Messwinkel von 60° bei Raumtemperatur durchgeführt. Der Glanzwert wird wie üblich dimensionslos als "Gloss Units" [GU] angegeben.

**Beispiel 3** - Herstellung der Zusammensetzungen für die Glanz-Beschichtung

Die verschiedenen Zusammensetzungen (Filmüberzugslösungen) wurden durch langsame Zugabe und vollständiges Lösen des Polyethylenglykols und des Polyvinylpyrrolidons (bzw. des Polyvinylpyrrolidon-Polyvinylacetat-Copolymers) in VE-Wasser hergestellt. Die Lösungen wurden während der Herstellung mit einem Magnetrührer gerührt. Eine Erwärmung der Lösung, während der Herstellung war nicht erforderlich. Der Polymergehalt der Zusammensetzungen (Filmüberzugslösungen) lag bevorzugt bei 10%. Es wurden verschiedene Polyethylenglykol und Polyvinylpyrrolidon (bzw. Polyvinylpyrrolidon Copolymer) in verschiedenen Mengenverhältnissen eingesetzt und getestet.

**Beispiel 4** - Auftragen der Zusammensetzung (des glanzgebenden Filmüberzugs)

Direkt nach dem Abkühlen der basisbeschichteten Filmtabletten aus Beispiel 2 wurden die nach Beispiel 3 hergestellten Zusammensetzungen (Filmüberzugslösungen) in der geeigneten Beschichtungs-Apparatur (24" Accela Cota) unter den unten aufgeführten Auftrags-Bedingungen aufgetragen. Es wurden direkt nach dem Auftragsprozess und nach dem Polierprozess mindestens 10 Tabletten als Proben gezogen, deren Glanzwert bestimmt wurde. Details zum Polierprozess sind ebenfalls unten aufgeführt.

Die Glanzmessung an den Filmtabletten wurde mit einem üblichen optischen Messgerät (Typ 400 Novo-Curve™) bei einem Messwinkel von 60° bei Raumtemperatur durchgeführt. Der Glanzwert wird wie üblich dimensionslos als "Gloss Units" [GU] angegeben.

| Bedingungen Auftragsprozess: | |
|---|---|
| Sprühdruck | 2,0 bar |
| Formierluftdruck | 1,0 bar |
| Kesseldrehzahl | 10 UpM |
| Zuluft-Menge | 60 L/s |
| Abluft-Menge | 110 L/s |
| Zuluft-Temperatur | 62°C |
| Abluft-Temperatur | 45°C |
| Produkttemperatur | 37°C |
| Sprührate | 10 g/min |
| Trocken-/Abkühlzeit | 5 min |
| Auftragsmenge | 0,5 mg/cm$^2$ |
| Batchgröße | 5 kg |

| Bedingungen Polierprozess: | |
|---|---|
| Kesseldrehzahl | 2-3 UpM |
| Zuluft-Menge | - |
| Abluft-Menge | - |
| Produkttempeartur | 37°C - 25°C |
| Prozessdauer | 30 min. |

**Beispiel 5** - Ausführliche Beschreibung eines Beispielprozesses

Für die Basis-Beschichtung wurde ein Kilogramm einer 20%igen Beschichtungslösung vom Typ III in VE-Wasser hergestellt.

Die entgaste PEG-PVA-Copolymer (Typ III) Lösung wurde in einer Tabletten-Beschichtungs-Apparatur (Horizontaltrommelcoater vom Typ 24" Accela Cota, Manesty) auf 5 kg Placebokerne (rund, gewölbt, Durchmesser 9 mm) folgender Zusammensetzung aufgesprüht:

| | |
|---|---|
| Ludipress LCE | 99,5 Gew.-% |
| Magnesium-Stearat | 0,5 Gew.-% |

| Sprühbedingungen: | |
|---|---|
| Sprühdruck : | 2,0 bar |
| Formierluftdruck : | 1,0 bar |
| Kesseldrehzahl : | 15 UpM |
| Zuluft-Menge : | 60 L/s |
| Abluft-Menge : | 110 L/s |
| Zuluft-Temperatur: | 62°C |
| Abluft-Temperatur: | 45°C |
| Produkttemperatur : | 37°C |
| Sprührate : | 25g/min |
| Trocken-/Abkühlzeit : | 5 min |
| Auftragsmenge : | 4,5 mg/cm$^2$ |
| Sprühzeit : | 28 min |

Nach der Basis-Beschichtung waren alle Tabletten mit einem gleichmäßigen, homogenen, carminfarbigen Film überzogen. Mindestens 10 Tabletten wurden als Probe für die Glanzmessung entnommen.

Die Beschichtungslösung (glanzgebende Filmüberzugslösung) bestand zu neun Teilen aus PEG 6000 und einem Teil Kollidon® 17, welche unter Rühren in 90 Teile Wasser eingearbeitet wurden. Die festen Substanzen wurden langsam zugegeben und waren nach etwa zehn Minuten vollständig gelöst.

Nach dem Trocknen der basisbeschichteten Tabletten in der Beschichtungs-Apparatur (24" Accela Cota) wurden 170 g der klaren Beschichtungslösung (glanzgebende Filmüberzugslösung) bei einer Kernbetttemperatur von ca. 36°C unter den unten angegebenen Bedingungen aufgetragen.

| Sprühbedingungen: | |
|---|---|
| Sprühdruck : | 2,0 bar |
| Formierluftdruck : | 1,0 bar |
| Kesseldrehzahl : | 15 UpM |
| Zuluft-Menge : | 60 L/s |
| Abluft-Menge : | 110 L/s |
| Zuluft-Temperatur: | 62°C |
| Abluft-Temperatur: | 45°C |
| Produkttemperatur : | 36°C |
| Sprührate : | 10g/min |
| Trocken-/Abkühlzeit : | 5 min |
| Auftragsmenge : | 0,5 mg/cm$^2$ |

Von den getrockneten, glanzüberzogenen Filmtabletten wird ebenfalls eine Probe von mindestens 10 Tabletten zur Glanzmessung entnommen. Die restlichen Filmtabletten werden bei niedriger Drehzahl in der Trommel unter den unten angegeben Bedingungen abgekühlt (Polierprozess).

| Polierbedingungen: | |
|---|---|
| Kesseldrehzahl : | 2-3 UpM |
| Zuluft-Menge : | - |
| Abluft-Menge : | - |
| Produkttemperatur : | 37°C - 25°C |

(fortgesetzt)

| Prozessdauer: | 30 min. |
|---|---|

Im Anschluss an die Polierphase werden die abgekühlten Filmtabletten aus der Trommel entnommen. Von zehn Tabletten wird der Glanzwert bestimmt.

Die Glanzmessung wurde wie in Beispiel 1 beschrieben durchgeführt.

**Beispiel 6** - Vergleich verschiedener Zusammensetzungen für die Glanz-Beschichtung

Für die Basis-Beschichtung wurde Lösung vom Typ II wie in Beispiel 1 beschrieben hergestellt und wie in Beispiel 2 beschrieben auf runde, gewölbte Placebokernen (99,5% Ludipress® LCE (BASF); 0,5% Mg- Stearat) mit einem Durchmesser von 9mm, aufgetragen.

Die Zusammensetzungen (Filmüberzugslösung) für die Glanzbeschichtung wurden wie in Beispiel 3 beschrieben hergestellt und die Beschichtung wurde wie in Beispiel 4 beschrieben durchgeführt, wobei jeweils die in den folgenden Tabellen 2 a) bis g) genannten PEG und PVP im angegebenen Verhältnis eingesetzt wurden.

Die Glanzmessung wurde wie in Beispiel 1 beschrieben durchgeführt. In den Tabellen 2a) - g) sind die Glanzwerte [GU] der entsprechenden Filmüberzugslösungen auf runden, gewölbten Placebokernen mit einem Durchmesser von 9mm, zusammengestellt.

Tabelle 2 a) Reine PEG - Lösung

|  | Ohne Polierschritt | Mit Polierschritt |
|---|---|---|
| PEG 2.000 | 5,9 | 6,8 |
| PEG 4.000 | 6,2 | 8,4 |
| PEG 6.000 | 8,0 | - |
| PEG 20.000 | 4,9 | 6,2 |

Tabelle 2 b) Reine Kollidon® - Lösungen

|  | Ohne Polierschritt | Mit Polierschritt |
|---|---|---|
| Kollidon® 17 | 3,1 | - |
| Kollidon® 30 | 4,4 | 4,8 |
| Kollidon® VA 64 | 3,5 | - |

Tabelle 2 c) PEG - Lösungen mit Kollidon® 12 (BASF)

|  | Verhältnis (PEG/PVP) 18:1 | | Verhältnis (PEG/PVP) 9:1 | | Verhältnis (PEG/PVP) 5:1 | |
|---|---|---|---|---|---|---|
|  | Ohne Polierschritt | Mit Polierschritt | Ohne Polierschritt | Mit Polierschritt | Ohne Polierschritt | Mit Polierschritt |
| PEG 1.500 | 7,4 | 8,2 | 8,1 | - | 7,2 | - |
| PEG 2.000 | 5,8 | 7,8 | 6,1 | 6,9 | 8,1 | 8,3 |
| PEG 4.000 | 6,3 | 7,6 | 8,6 | 8,6 | 6,0 | - |
| PEG 6.000 | 10,1 | - | 11,0 | 11,8 | 10,0 | 10,7 |

(fortgesetzt)

| | Verhältnis (PEG/PVP) 18:1 | | Verhältnis (PEG/PVP) 9:1 | | Verhältnis (PEG/PVP) 5:1 | |
|---|---|---|---|---|---|---|
| | Ohne Polierschritt | Mit Polierschritt | Ohne Polierschritt | Mit Polierschritt | Ohne Polierschritt | Mit Polierschritt |
| PEG 20.000 | 4,9 | 5,8 | 3,9 | 5,1 | 6,9 | 7,9 |

Tabelle 2 d) PEG - Lösungen mit Kollidon® 17 (BASF)

| | Verhältnis (PEG/PVP) 18:1 | | Verhältnis (PEG/PVP) 9:1 | | Verhältnis (PEG/PVP) 5:1 | |
|---|---|---|---|---|---|---|
| | Ohne Polierschritt | Mit Polierschritt | Ohne Polierschritt | Mit Polierschritt | Ohne Polierschritt | Mit Polierschritt |
| PEG 1.500 | 8,0 | 8,4 | 8,6 | 8,8 | 8,0 | 7,2 |
| PEG 2.000 | 7,9 | 8,2 | 9,2 | 10,1 | 6,8 | 7,4 |
| PEG 4.000 | 9,5 | - | 10,6 | - | 10,0 | 7,9 |
| PEG 6.000 | 8,7 | 9,9 | 11,9 | 12,3 | 10,9 | 9,8 |
| PEG 20.000 | - | - | 6,8 | 8,9 | - | - |

Tabelle 2 e) PEG - Lösungen mit Kollidon® 30 (BASF)

| | Verhältnis (PEG/PVP) 18:1 | | Verhältnis (PEG/PVP) 9:1 | | Verhältnis (PEG/PVP) 5:1 | |
|---|---|---|---|---|---|---|
| | Ohne Polierschritt | Mit Polierschritt | Ohne Polierschritt | Mit Polierschritt | Ohne Polierschritt | Mit Polierschritt |
| PEG 1.500 | 7,9 | - | 9,6 | 9,8 | 8,0 | - |
| PEG 2.000 | - | - | 8,4 | - | - | - |
| PEG 4.000 | 9,8 | 10,6 | 8,1 | 8,4 | 9,0 | - |
| PEG 6.000 | 8,1 | 9,4 | 9,1 | 10,1 | 8,2 | 8,7 |
| PEG 20.000 | 6,2 | 7,3 | 5,9 | 6,8 | 7,4 | 8,2 |

Tabelle 2 f) PEG - Lösungen mit Kollidon® 90 (BASF)

| | Verhältnis (PEG/PVP) 18:1 | | Verhältnis (PEG/PVP) 9:1 | | Verhältnis (PEG/PVP) 5:1 | |
|---|---|---|---|---|---|---|
| | Ohne Polierschritt | Mit Polierschritt | Ohne Polierschritt | Mit Polierschritt | Ohne Polierschritt | Mit Polierschritt |
| PEG 1.500 | - | - | 12,1 | - | - | - |
| PEG 2.000 | - | - | 10,0 | - | - | - |
| PEG 4.000 | 8,0 | 8,6 | 10,3 | 11,6 | 9,2 | - |
| PEG 6.000 | 10,1 | - | 9,7 | - | 9,0 | - |
| PEG 20.000 | 4,9 | 5,4 | 8,0 | - | 5,9 | 8,0 |

Tabelle 2 g) PEG - Lösungen mit Kollidon® VA 64 (BASF)

| | Verhältnis (PEG/PVP) 18:1 | | Verhältnis (PEG/PVP) 9:1 | | Verhältnis (PEG/PVP) 5:1 | |
|---|---|---|---|---|---|---|
| | Ohne Polierschritt | Mit Polierschritt | Ohne Polierschritt | Mit Polierschritt | Ohne Polierschritt | Mit Polierschritt |
| PEG 1.500 | - | - | 6,4 | - | - | - |
| PEG 2.000 | - | - | 3,8 | 7,6 | - | - |
| PEG 4.000 | 6,2 | 7,9 | 9,3 | - | 9,1 | 10,4 |
| PEG 6.000 | 7,7 | 7,6 | 10,2 | 10,3 | 8,9 | 9,6 |
| PEG 20.000 | - | - | 6,7 | 8,1 | - | - |

Das Auftragen einer wässrigen Lösung aus PEG und PVP bewirkt in jedem Fall eine deutliche Glanzverbesserung. Das gilt für alle getesteten Kombinationen. Die Kombinationen aus PVP und PEG bewirken eine deutlich größere Glanzverbesserung als Lösungen der Einzelkomponente. Die Nachbehandlung (Polieren) bei niedriger Drehzahl und geringer Zuluft-Temperatur bewirkt bei einigen Zusammensetzungen eine Verbesserung des Glanzes. Die Zusammensetzung für die Glanz-Beschichtung kann direkt nach der Basis-Beschichtung aufgetragen werden. Es ist keine zusätzliche Vorrichtung erforderlich und die Applikationsdauer ist im Vergleich zum Basis-Beschichtungsprozess kurz.

**Beispiel 7:** Vergleich der Glanzbeschichtung auf verschiedenen Farben und Basisbeschichtungen

Für die Basis-Beschichtungen wurden Beschichtungslösungen vom Typ I - VIII wie in Beispiel 1 beschrieben hergestellt. Es wurden Beschichtungen auf runde, gewölbte Placebokernen (99,5% Ludipress® LCE (BASF); 0,5% Mg-Stearat) mit einem Durchmesser von 9mm wie in Beispiel 2 beschrieben durchgeführt.
Die Zusammensetzungen für den Glanzüberzug wurden wie in Beispiel 3 beschrieben aus 9 Teilen PEG 6000, einem Teil PVP Kollidon® 17 (BASF) und 90 Teilen VE-Wasser hergestellt. Die Beschichtungslösung wurde wie in Beispiel 4 beschrieben auf die oben beschriebenen basisbeschichteten Tabletten aufgetragen.
Die Glanzmessung wurde wie in Beispiel 1 beschrieben durchgeführt. Die Glanzwerte der beschichteten Tabletten sind in Tabelle 3 angegeben.

Tabelle 3: Glanzüberzüge auf Basis-Beschichtungen verschiedener Farben und Polymeren

| Basis-Beschichtung | Glanzwert der Basis-Beschichtung | Glanzwert ohne Polierschritt | Glanzsteigerung [%] | Glanz-wert mit Polierschritt | Glanzsteigerung [%] |
|---|---|---|---|---|---|
| Typ I | 4,4 | 9,8 | 124,3 | 11,1 | 153,1 |
| Typ II | 2,8 | 10,8 | 285,7 | 13,2 | 371,4 |
| Typ III | 4,0 | 10,2 | 157,1 | - | - |
| Typ IV | 2,5 | 8,2 | 230,6 | - | - |
| Typ V | 3,0 | 9,3 | 206,3 | - | - |
| Typ VI | 2,9 | 9,1 | 218,1 | - | - |
| Typ VII | 4,0 | 11,3 | 182,5 | - | - |
| Typ VIII | 4,2 | 10,1 | 140,5 | 10,3 | 145,2 |

Das Auftragen einer wässrigen Zusammensetzung (Filmüberzugslösung) aus PEG und PVP bewirkt in jedem Fall eine deutliche Glanzverbesserung. Das gilt für alle getesteten Kombinationen anwendbar, der zur Herstellung verwendeten Stoffe. Die Lösung ist auf allen getesteten Farben und auf allen getesteten filmbildenden Polymeren anwendbar und bewirkt eine extrem starke Glanzsteigerung von bis zu 371,4 %. Die Nachbehandlung (Polieren) bei niedriger Drehzahl und geringer Zuluft-Temperatur bewirkt bei einigen Zusammensetzungen eine zusätzliche Verbesserung des Glanzes.

**Beispiel 8:** Vergleich der Glanz-Beschichtung auf verschiedenen Tablettenformen

Für die Basis-Beschichtung wurde eine Beschichtungslösung Typ II wie in Beispiel 1 beschrieben hergestellt und auf Placebokerne aus 99,5 Gew.-% Ludipress® LCE (BASF) und 0,5 Gew.-% Mg- Stearat wie in Beispiel 2 beschrieben auf folgende Tablettenformen aufgetragen:

- rund, gewölbt, 6mm,
- rund, gewölbt, 9mm
- oblong 8,5mm

Die Zusammensetzungen für den Glanzüberzug wurden wie in Beispiel 3 beschrieben aus 9 Teilen PEG 6000, einem Teil PVP Kollidon® 17 (BASF) und 90 Teilen Wasser hergestellt. Die Beschichtungslösung wurde wie in Beispiel 4 beschrieben auf die oben beschriebenen basisbeschichteten Tabletten aufgetragen.
Die Glanzmessung wurde wie in Beispiel 1 beschrieben durchgeführt. In Tabelle 5 sind die Glanzwerte der beschichteten Tabletten angegeben.

Tabelle 5: Glanzbeschichtungen auf verschiedenen Tablettenformen

| | rund, gewölbt, 6mm | | rund, gewölbt, 9mm | | oblong 8,5mm | |
|---|---|---|---|---|---|---|
| | Ohne Polierschritt | Mit Polierschritt | Ohne Polierschritt | Mit Polierschritt | Ohne Polierschritt | Mit Polierschritt |
| Ohne Glanzbeschichtung | Visuell starke Glanzverbesserung | - | 2,8 | - | 3,2 | - |
| PEG 6000/ Kollidon® 17 (9:1) | Visuell starke Glanzverbesserung | Visuell starke Glanzverbesserung | 10,8 | 13,2 | 10,3 | 11,8 |

Das Auftragen einer glanzgebenden Filmüberzugslösung aus PEG und PVP bewirkt in jedem Fall eine deutliche Glanzverbesserung. Die Lösung ist auf allen getesteten Tab lettenformen anwendbar und bewirkt eine starke Glanzsteigerung. Die Nachbehandlung (Polieren) bei niedriger Drehzahl und geringer Zuluft-Temperatur bewirkt eine zusätzliche Verbesserung des Glanzes.

**Patentansprüche**

1.   Zusammensetzung zur Beschichtung von festen Substraten (S) bestehend aus:

   a) 5-20 Gew.-% eines wasserlöslichen Polyethylenglykol mit einem gewichtsmittleren Molekulargewicht im Bereich von 1.500 bis 10.000 g/mol (Komponente A),
   b) 0,5-5 Gew.-% eines wasserlöslichen Polyvinylpyrrolidons mit einem K-Wert nach Fikentscher im Bereich von 12 bis 90 (Komponente B),
   c) 65-95 Gew.-% Wasser als Lösemittel (L), sowie
   d) 0-10 Gew.-% eines oder mehrerer nicht polymerer Hilfsmittel (Komponente C),

   wobei es sich bei der Zusammensetzung um eine wässrige Lösung des Polyethylenglykol, des Polyvinylpyrrolidons und optional der nicht polymeren Hilfsmittel handelt,
   und wobei die Zusammensetzung einen Polymergehalt im Bereich von 5 bis 20 Gew.-% aufweist und Polyethylenglykol und Polyvinylpyrrolidon in einem Gewichtsverhältnis von 15:1 bis 7:1 vorliegen.

2.   Zusammensetzung zur Beschichtung von festen Substraten nach Anspruch 1 bestehend aus:

   a) 5-20 Gew.-% eines wasserlöslichen Polyethylenglykol mit einem gewichtsmittleren Molekulargewicht im Bereich von 4.000 bis 10.000 g/mol (Komponente A)
   b) 0,5-5 Gew.-% eines wasserlöslichen Polyvinylpyrrolidons mit einem K-Wert nach Fikentscher im Bereich von 12 bis 30 (Komponente B)
   c) 75-95 Gew.-% Wasser als Lösemittel (L).

3.   Zusammensetzung zur Beschichtung von festen Substraten nach einem der Ansprüche 1 oder 2, **dadurch gekennzeichnet, dass** Komponente B ausgewählt ist aus einem oder mehreren Polyvinylpyrrolidonen mit einem K-Wert von 12, 17, 30, 90.

4.   Zusammensetzung zur Beschichtung von festen Substraten nach einem der Ansprüche 1 bis 3, **dadurch gekennzeichnet, dass** die Lösung einen Polymergehalt im Bereich von 5 bis 20 Gew.-% aufweist und Polyethylenglykol und Polyvinylpyrrolidon in einem Gewichtsverhältnis von etwa 9:1 vorliegen.

5.   Zusammensetzung zur Beschichtung von festen Substraten nach einem der Ansprüchen 1 bis 4, **dadurch gekennzeichnet, dass** sie besteht aus:

   a) 8-10 Gew.-% eines wasserlöslichen Polyethylenglykol mit einem gewichtsmittleren Molekulargewicht von 6000 g/mol (PEG 6000) (Komponente A)
   b) 0,5-2 Gew.-% eines wasserlöslichen Polyvinylpyrrolidons mit einem K-Wert nach Fikentscher von 17 (Komponente B)
   c) 88-95 Gew.-% Wasser.

6.   Verfahren zur Herstellung beschichteter fester Substrate, bei dem man auf ein festes Substrat (S) mindestens eine Zusammensetzung gemäß einem der Ansprüche 1 bis 5 aufbringt.

7.   Verfahren zur Herstellung beschichteter fester Substrate nach Anspruch 6, enthaltend die folgenden Schritte:

   a) Herstellen einer Beschichtungslösung durch Vermischen der Komponenten A und B und des Lösemittels L,
   b) Aufbringen der Beschichtungslösung mittels eines geeigneten Verfahrens auf das Substrat (S),
   c) gegebenenfalls Trocknen des beschichteten Substrats durch das Aufbringen von Gas bei einer Temperatur im Bereich von 20°C bis 80°C, sowie
   d) gegebenenfalls Polieren des beschichteten und getrockneten Substrats.

8. Verfahren zur Herstellung beschichteter fester Substrate gemäß einem der Ansprüche 6 oder 7, **dadurch gekennzeichnet, dass** das Substrat (S) ausgewählt ist aus: festen pharmazeutischen, kosmetischen und agrochemischen Darreichungsformen, Saatgut, Nahrungsergänzungsmitteln und Lebensmitteln.

9. Verfahren zur Herstellung beschichteter fester Substrate gemäß einem der Ansprüche 6 bis 8, **dadurch gekennzeichnet, dass** das Substrat (S) aus einer festen pharmazeutischen Darreichungsform besteht, welche bereits mit mindestens einer polymeren Filmbeschichtung versehen ist.

10. Beschichtetes Substrat, das beschichtet ist mit einer Zusammensetzung nach einem der Ansprüche 1 bis 5.

11. Beschichtetes Substrat nach Anspruch 10, wobei das Substrat (S) ausgewählt ist aus pharmazeutischen, kosmetischen und agrochemischen Darreichungsformen, Saatgut, Nahrungsergänzungsmitteln und Lebensmitteln.

**Claims**

1. A composition for coating solid substrates (S) consisting of:

   a) 5-20% by weight of a water-soluble polyethylene glycol with a weight-average molecular weight in the range from 1500 to 10 000 g/mol (component A),
   b) 0.5-5% by weight of a water-soluble polyvinylpyrrolidone with a Fikentscher K value in the range from 12 to 90 (component B),
   c) 65-95% by weight of water as solvent (L), and
   d) 0-10% by weight of one or more non-polymeric aids (component C),

   where the composition is an aqueous solution of the polyethylene glycol, of the polyvinylpyrrolidone and optionally of the non-polymeric aids,
   and where the composition has a polymer content in the range from 5 to 20% by weight and polyethylene glycol and polyvinylpyrrolidone are present in a weight ratio of 15:1 to 7:1.

2. The composition for coating solid substrates according to claim 1 consisting of:

   a) 5-20% by weight of a water-soluble polyethylene glycol with a weight-average molecular weight in the range from 4000 to 10 000 g/mol (component A),
   b) 0.5-5% by weight of a water-soluble polyvinylpyrrolidone with a Fikentscher K value in the range from 12 to 30 (component B),
   c) 75-95% by weight of water as solvent (L).

3. The composition for coating solid substrates according to either of claims 1 and 2, wherein component B is selected from one or more polyvinylpyrrolidones with a K value of 12, 17, 30, 90.

4. The composition for coating solid substrates according to any of claims 1 to 3, wherein the solution has a polymer content in the range from 5 to 20% by weight, and polyethylene glycol and polyvinylpyrrolidone are present in a ratio of about 9:1 by weight.

5. The composition for coating solid substrates according to any of claims 1 to 4, which consists of:

   a) 8-10% by weight of a water-soluble polyethylene glycol with a weight-average molecular weight of 6000 g/mol (PEG 6000) (component A),
   b) 0.5-2% by weight of a water-soluble polyvinylpyrrolidone with a Fikentscher K value of 17 (component B), and
   c) 88-95% by weight of water.

6. A process for producing coated solid substrates in which at least one composition according to any of claims 1 to 5 is applied to a solid substrate (S).

7. The process for producing coated solid substrates according to claim 6, comprising the following steps:

   a) preparing a coating solution by mixing components A and B and the solvent L.

b) applying the coating solution by means of a suitable process to the substrate (S),
c) optionally drying the coated substrate by applying gas at a temperature in the range from 20°C to 80°C, and
d) optionally polishing the coated and dried substrate.

8. The process for producing coated solid substrates according to either of claims 6 and 7, wherein the substrate (S) is selected from: solid pharmaceutical, cosmetic and agrochemical delivery forms, seeds, dietary supplements and food products.

9. The process for producing coated solid substrates according to any of claims 6 to 8, wherein the substrate (S) consists of a solid pharmaceutical dosage form which is already provided with at least one polymeric film coating.

10. A coated substrate which is coated with a composition according to any of claims 1 to 5.

11. The coated substrate according to claim 10, wherein the substrate (S) is selected from pharmaceutical, cosmetic and agrochemical delivery forms, seeds, dietary supplements and food products.

**Revendications**

1. Composition pour le revêtement de substrats solides (S), constituée par :

a) 5 à 20 % en poids d'un polyéthylène glycol soluble dans l'eau ayant un poids moléculaire moyen en poids dans la plage allant de 1 500 à 10 000 g/mol (composant A),
b) 0,5 à 5 % en poids d'une polyvinylpyrrolidone soluble dans l'eau ayant une valeur K selon Fikentscher dans la plage allant de 12 à 90 (composant B),
c) 65 à 95 % en poids d'eau en tant que solvant (L), et
d) 0 à 10 % en poids d'un ou de plusieurs adjuvants non polymères (composant C),

la composition étant une solution aqueuse du polyéthylène glycol, de la polyvinylpyrrolidone et éventuellement de l'adjuvant non polymère,
et la composition présentant une teneur en polymères dans la plage allant de 5 à 20 % en poids, et le polyéthylène glycol et la polyvinylpyrrolidone étant présents en un rapport en poids de 15:1 à 7:1.

2. Composition pour le revêtement de substrats solides selon la revendication 1, constituée par :

a) 5 à 20 % en poids d'un polyéthylène glycol soluble dans l'eau ayant un poids moléculaire moyen en poids dans la plage allant de 4 000 à 10 000 g/mol (composant A),
b) 0,5 à 5 % en poids d'une polyvinylpyrrolidone soluble dans l'eau ayant une valeur K selon Fikentscher dans la plage allant de 12 à 30 (composant B),
c) 75 à 95 % en poids d'eau en tant que solvant (L).

3. Composition pour le revêtement de substrats solides selon l'une quelconque des revendications 1 ou 2, **caractérisée en ce que** le composant B est choisi parmi une ou plusieurs polyvinylpyrrolidones ayant une valeur K de 12, 17, 30, 90.

4. Composition pour le revêtement de substrats solides selon l'une quelconque des revendications 1 à 3, **caractérisée en ce que** la solution présente une teneur en polymères dans la plage allant de 5 à 20 % en poids et le polyéthylène glycol et la polyvinylpyrrolidone sont présents en un rapport en poids d'environ 9:1.

5. Composition pour le revêtement de substrats solides selon l'une quelconque des revendications 1 à 4, **caractérisée en ce qu'**elle est constituée par :

a) 8 à 10 % en poids d'un polyéthylène glycol soluble dans l'eau ayant un poids moléculaire moyen en poids de 6 000 g/mol (PEG 6000) (composant A),
b) 0,5 à 2 % en poids d'une polyvinylpyrrolidone soluble dans l'eau ayant une valeur K selon Fikentscher de 17 (composant B),
c) 88 à 95 % en poids d'eau.

6. Procédé de fabrication de substrats solides revêtus, selon lequel au moins une composition selon l'une quelconque des revendications 1 à 5 est appliquée sur un substrat solide (S).

7. Procédé de fabrication de substrats solides revêtus selon la revendication 6, contenant les étapes suivantes :

a) la fabrication d'une solution de revêtement par mélange des composants A et B et du solvant L,
b) l'application de la solution de revêtement par un procédé approprié sur le substrat (S),
c) éventuellement le séchage du substrat revêtu par l'application d'un gaz à une température dans la plage allant de 20 °C à 80 °C, et
d) éventuellement le polissage du substrat revêtu et séché.

8. Procédé de fabrication de substrats solides revêtus selon l'une quelconque des revendications 6 ou 7, **caractérisé en ce que** le substrat (S) est choisi parmi : les formes médicamenteuses pharmaceutiques, cosmétiques et agrochimiques, les graines, les compléments alimentaires et les produits alimentaires.

9. Procédé de fabrication de substrats solides revêtus selon l'une quelconque des revendications 6 à 8, **caractérisé en ce que** le substrat (S) est constitué par une forme médicamenteuse pharmaceutique qui est déjà munie d'au moins un film de revêtement polymère.

10. Substrat revêtu, qui est revêtu avec une composition selon l'une quelconque des revendications 1 à 5.

11. Substrat revêtu selon la revendication 10, dans lequel le substrat (S) est choisi parmi les formes médicamenteuses pharmaceutiques, cosmétiques et agrochimiques, les graines, les compléments alimentaires et les produits alimentaires.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- GB 1021178 A
- US 4060598 A
- WO 2006102446 A
- EP 0572942 A
- EP 1232747 A
- EP 1929997 A
- US 20080031950 A